# EUROPEAN PATENT APPLICATION

(11) **EP 3 961 643 A1**
(43) Date of publication of application: **02.03.2022**
(21) Application number: 21192831.2
(22) Date of filing: 24.08.2021
(51) Int. Cl.: G16H 20/30, G16H 50/70, G16H 70/20, A61B 5/00, A63B 24/00

(54) **TRAINING SYSTEM, TRAINING METHOD, AND PROGRAM**

(30) Priority: 26.08.2020 JP 2020142646
(71) Applicant: TOYOTA JIDOSHA KABUSHIKI KAISHA, Toyota-shi, Aichi-ken, 471-8571 (JP)
(72) Inventor: IWATA, Takuya, Aichi-ken, 471-8571 (JP); MIYAGAWA, Toru, Aichi-ken, 471-8571 (JP); NAKASHIMA, Issei, Aichi-ken, 471-8571 (JP); KOBAYASHI, Makoto, Aichi-ken, 471-8571 (JP); SUGA, Keisuke, Aichi-ken, 471-8571 (JP); IMAIDA, Masayuki, Aichi-ken, 471-8571 (JP); YUASA, Hisataka, Aichi-ken, 471-8571 (JP); SAITOH, Eiichi, Aichi-ken, 471-8571 (JP); OTAKA, Yohei, Aichi-ken, 471-8571 (JP); HIRANO, Satoshi, Aichi-ken, 471-8571 (JP)
(74) Representative: D Young & Co LLP

(57) **Abstract**

A training system (10) generates a training plan for enabling a trainee to perform a plurality of kinds of training, and includes: a track record information accepting unit (12) that acquires track record information regarding the physical action of the trainee; the target information generation unit (11) that accepts environmental data of daily life of the trainee after the training plan is finished, and generates target information regarding a target of the function recovery of the trainee based on the accepted environmental data; the storage unit (14) that stores statistical information regarding the function recovery in the training performed by a plurality of other trainees; and the plan generation unit (13) that generates the training plan of the trainee based on the track record information, the target information, and the statistical information and outputs the generated training plan.

## Description

### BACKGROUND

The present disclosure relates to a training system, a training method, and a program.

Training systems for the purpose of recovering functions of physical actions of trainees have been developed. For example, a walking training apparatus according to Japanese Unexamined Patent Application Publication No. 2015-223294 includes a walking assist device, a first tensile means, and a second tensile means. The walking assist device, which is mounted on a leg part of a trainee, assists walking of the trainee. The first tensile means pulls at least one of the walking assist device and the leg part of the trainee upward and frontward. The second tensile means pulls at least one of the walking assist device and the leg part of the trainee upward and rearward.

### SUMMARY

When the training system described above is used, a manager who manages a training plan manually generates or updates the training plan based on his/her experience and intuition. However, there has been a demand to implement a system capable of generating an efficient training plan for a set target.

The present disclosure has been made in order to solve the above problem, and an object thereof is to provide a training system and the like that enable a trainee to efficiently perform function recovery training for a set target.

A first exemplary aspect is a training system configured to generate a training plan for enabling a trainee to perform a plurality of kinds of training for the purpose of recovering a function of a physical action of the trainee, and the training system includes a track record information accepting unit, a target information generation unit, a storage unit, and a plan generation unit. The track record information accepting unit acquires track record information regarding the physical action of the trainee. The target information generation unit accepts environmental data of daily life of the trainee after the training plan is finished, and generates target information regarding a target of the function recovery of the trainee based on the accepted environmental data. The storage unit stores statistical information regarding the function recovery in the training performed by a plurality of other trainees. The plan generation unit generates the training plan of the trainee based on the track record information, the target information, and the statistical information and outputs the generated training plan.

The above configuration enables the training system to set a target in accordance with an environment after the trainee finishes training, and output the training plan for the set target.

In the aforementioned training system, the target information generation unit may acquire, by it being connected to a management apparatus configured to manage the environmental data, the environmental data from the management apparatus. This configuration enables the training system to appropriately acquire desired information.

In the aforementioned training system, the storage unit may include, in the environmental data, data regarding a tool used by the trainee in the physical action, and the target information generation unit may present a candidate for the tool to be used by the trainee after the training plan is finished based on the environmental data. Further, in the aforementioned training system, the plan generation unit may determine the tool used by the trainee in the training plan based on the tool to be used by the trainee after the training plan is finished. This configuration enables the training system to output the training plan in accordance with the tool to be used by the trainee after the training plan is finished.

In the aforementioned training system, the track record information accepting unit may acquire, as the track record information, information from a sensor that has detected the physical action of the trainee. Further, in the aforementioned training system, the track record information accepting unit may acquire, as the track record information, image information from a camera that has captured images of the physical action of the trainee. This configuration enables the training system to appropriately acquire desired track record information.

In the aforementioned training system, the track record information accepting unit may acquire information regarding an action level of the trainee set using at least information regarding a type of the tool used by the trainee in the physical action and a level of achievement of the physical action. Further, in the aforementioned training system, the plan generation unit may generate the training plan based on the action level. Further, the target information generation unit may generate the action level as the target information based on the environmental data. This configuration enables the training system to output a training plan using an index which a user can easily understand.

Another exemplary aspect is a training method for generating a training plan for enabling a trainee to perform a plurality of kinds of training for the purpose of recovering a function of a physical action of the trainee, and the training method includes a track record information accepting step, a target information generation step, a storage step, and a plan generation step. The track record information accepting step acquires track record information regarding the physical action of the trainee. The target information generation step accepts environmental data of daily life of the trainee after the training plan is finished, and generating target information regarding a target of the function recovery of the trainee based on the accepted environmental data. The storage step stores statistical information regarding the function recovery in the training performed by a plurality of other trainees. The plan generation step generates the training plan of the trainee based on the track record information, the target information, and the statistical information and outputs the generated training plan.

The above configuration enables the training system to set a target in accordance with an environment after the trainee finishes training, and output the training plan for the set target.

Another exemplary aspect is a program for causing a computer to execute a training method for generating a training plan for enabling a trainee to perform a plurality of kinds of training for the purpose of recovering a function of a physical action of the trainee. The aforementioned training method includes a track record information accepting step, a target information generation step, a storage step, and a plan generation step. The track record information accepting step acquires track record information regarding the physical action of the trainee. The target information generation step accepts environmental data of daily life of the trainee after the training plan is finished, and generating target information regarding a target of the function recovery of the trainee based on the accepted environmental data. The storage step stores statistical information regarding the function recovery in the training performed by a plurality of other trainees. The plan generation step generates the training plan of the trainee based on the track record information, the target information, and the statistical information and outputs the generated training plan.

The above configuration enables the training system to set a target in accordance with an environment after the trainee finishes training, and output the training plan for the set target.

According to the present disclosure, it is possible to provide a training system and the like that enable a trainee to perform function recovery training efficiently.

The above and other objects, features and advantages of the present disclosure will become more fully understood from the detailed description given hereinbelow and the accompanying drawings which are given by way of illustration only, and thus are not to be considered as limiting the present disclosure.

### BRIEF DESCRIPTION OF DRAWINGS

Fig. 1 is a block diagram of a training system according to a first embodiment;
Fig. 2 is a flowchart of the training system according to the first embodiment;
Fig. 3 is a diagram showing training information according to the first embodiment;
Fig. 4 is a diagram showing an action level according to the first embodiment;
Fig. 5 is a diagram showing statistical information according to the first embodiment;
Fig. 6 is a diagram showing an example of environmental data according to the first embodiment;
Fig. 7 is a diagram showing an example of target information generated from an accepted environmental data;
Fig. 8 is a diagram showing track record information and the target information of the training system according to the first embodiment;
Fig. 9 is a diagram showing an example of a training plan according to the first embodiment; and
Fig. 10 is a block diagram of a training system according to a second embodiment.

### DESCRIPTION OF EMBODIMENTS

Hereinafter, the present disclosure will be explained with reference to embodiments of the present disclosure. However, the disclosure set forth in the claims is not limited to the following embodiments. Further, not all the structures explained in the embodiments may be necessary as means for solving the problem. For a purpose of clarifying the description, the following description and the drawings will be omitted and simplified as appropriate. Throughout the drawings, the same components are denoted by the same reference symbols and overlapping descriptions will be omitted as necessary.

### <Embodiments>

Hereinafter, with reference to the drawings, embodiments of the present disclosure will be described. A training system according to the embodiments generates a training plan for enabling a trainee to carry out a plurality of kinds of training for the purpose of recovering functions of his/her impaired physical actions. The trainee here is, for example, a person suffering from paralysis in a part of his/her body, which is caused by a stroke (cerebrovascular disease) or a spinal cord injury. The trainee's physical action functions have been impaired due to the above disease. In this embodiment, physical actions are, for example, actions included in Activities of Daily Living (ADL) and include actions such as eating, toileting, dressing, walking, etc.

A trainee carries out a plurality of kinds of training in order to recover the functions of his/her impaired physical actions. To be specific, for example, training for recovering functions of a physical action eating includes training of a meal intake action, training of a swallowing action, and the like. Further, the training for recovering functions of the toileting action includes, for example, training of a moving action for moving to each of a bed, a wheelchair, and a toilet seat, training for toileting, and the like. Further, the training for recovering the function of the walking action includes, for example, training for maintaining the sitting posture, training for maintaining the standing posture, training of flat ground walking, training of walking on a slope, and the like. Further, the training includes, besides the training in which the trainee actively carries out physical actions as described above, passive training such as a massage in which the trainee receives stimulation to his/her joints or muscles from an assistant. The aforementioned training is also referred to as rehabilitation.

Some training performed using the training system may require assistance by therapists. Therapists are persons who have expertise that is necessary to assist trainees. In this embodiment, the therapists include a physical therapist, an occupational therapist, and a speech therapist. The therapists assist training performed by the trainees in accordance with expertise that they each have.

The physical therapist is a person who offers a physical therapy for persons who have physical disabilities mainly for the purpose of recovering fundamental action abilities. The physical therapy is a therapeutic treatment performed on persons whose functions of physical actions have been impaired due to diseases, injuries, disabilities and the like for the purpose of maintaining or improving these functions. The physical therapist carries out a physical therapy by using exercises, heat, electricity and the like. The physical therapist is also referred to as a Physical Therapist (PT).

The occupational therapist is a person who offers an occupational therapy for persons who have physical or mental disabilities mainly for the purpose of recovering their applicative action abilities or social adaptability. The "occupation" here includes daily activities such as bathing, dressing, and toileting. The occupational therapist is also referred to as OT.

The speech therapist is a person who carries out language training and other training exercises and provides tests, advice, guidance, and other assistance necessary for the above training exercises for persons who have disabilities in their speech functions, language functions, or hearing for the purpose of maintaining and improving these functions. The speech therapist further conducts training such as meal intake or swallowing under the instructions of doctors or dentists. The speech therapist is also referred to as a speech-language-hearing therapist or ST.

### <First Embodiment>

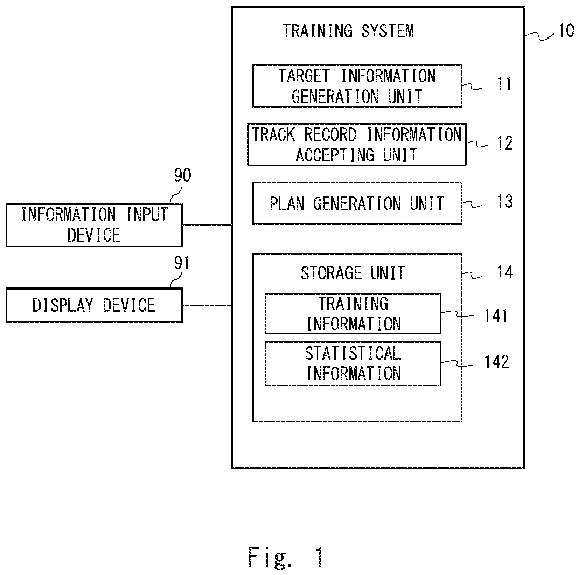

Hereinafter, a first embodiment will be described. Fig. 1 is a block diagram of a training system according to the first embodiment. A training system 10 shown in Fig. 1 includes, for example, a flash memory, a circuit board on which a Dynamic Random Access Memory (DRAM), a Central Processing Unit (CPU) and the like are mounted, and executes a control program stored in the memory, thereby implementing the function of the system. Further, the training system 10 is not necessarily implemented by software executed by a control program stored in advance in a non-volatile memory, and may instead be implemented, for example, by any combination of hardware, firmware, and software.

The training system 10 mainly includes a target information generation unit 11, a track record information accepting unit 12, a plan generation unit 13, and a storage unit 14. Further, these components included in the training system 10 are connected to one another as appropriate in such a way that they can communicate with one another. The training system 10 is further connected to an information input device 90 and a display device 91 in such a way that they can communicate with each other.

Hereinafter, main components of the training system 10 will be described. The target information generation unit 11 accepts environmental data of daily life of a trainee after a training plan is finished. The environmental data accepted by the target information generation unit 11 is, for example, data indicating an environment when the trainee has finished the training plan, left the hospital where he/she has been hospitalized, and returned home. Specific examples of the environmental data include data indicating whether or not the trainee lives at home with an assistant, whether or not the trainee goes up and down stairs, whether or not the trainee has a handrail for walking assistance at home, and the like. The specific examples of environmental data may also include data indicating what activities the trainee performs in his/her daily life. Specific examples of the environmental data in this case include data indicating whether or not the trainee uses public transportation, whether or not the trainee crosses streets at traffic lights, whether or not the trainee crosses pedestrian bridges, and the like. The target information generation unit 11 accepts the above-described environmental data via an information input device 90.

When the target information generation unit 11 accepts environmental data, it generates target information from the accepted environmental data. The target information is information regarding a target of the function recovery of the trainee, and is information serving as a target of the plan in the training plan generated by the training system 10. That is, the target information includes the content of training to be carried out by the trainee and a level of achievement of the training. The target information generation unit 11 converts environmental data into target information by associating the environmental data accepted via the information input device 90 with the target information. The target information generation unit 11 may store in advance information for associating the environmental data with the target information. The target information generation unit 11 supplies the generated target information to the plan generation unit 13.

The storage unit 14 may include data regarding a tool used by the trainee in the physical action in the environmental data, and the target information generation unit 11 may present a candidate for the tool to be used by the trainee after the training plan is finished from the data regarding the tool.

The track record information accepting unit 12 is an interface that acquires information supplied from the information input device 90. The track record information accepting unit 12 acquires the track record information regarding the physical actions of a trainee. The track record information includes information regarding the physical actions of a trainee for whom a training plan is to be generated and includes, for example, information indicating which kind of action the trainee may perform using which tool. The track record information may be indicated by a preset index. The track record information accepting unit 12 supplies the acquired track record information to the plan generation unit 13.

The plan generation unit 13 generates, when it has accepted the target information and the track record information, a training plan in accordance with the accepted target information and track record information and outputs the generated training plan. The training plan includes, for example, the content of the training performed by the trainee, the type of a therapist who is in charge of this training, the tools used in this training, and an execution period of the training. The plan generation unit 13 generates this training plan by using training information 141 and statistical information 142. The plan generation unit 13 outputs the generated training plan to the display device 91.

The plan generation unit 13 may refer to the target information generated from the tool to be used by the trainee after the training plan is finished, and determine the tool used by the trainee in the training plan. This configuration enables the training system to output the training plan in accordance with the tool to be used by the trainee after the training plan is finished.

The storage unit 14 includes a non-volatile memory such as a flash memory, an Erasable Programmable Read Only Memory (EPROM), or a Solid State Drive (SSD). The storage unit 14 stores the training information 141 and the statistical information 142.

The training information 141 includes the content of a plurality of kinds of training. The training information 141 includes information regarding the type of a therapist who is in charge of training and assists a trainee. The training information 141 also includes information regarding the type of the tool used in the training.

The statistical information 142 is information regarding the function recovery in training exercises that a plurality of other trainees have performed. When the training plan is generated, the storage unit 14 stores the aforementioned information in advance. Then the storage unit 14 supplies the aforementioned information to the plan generation unit 13 as appropriate.

Next, a structure connected to the training system 10 will be described. The information input device 90 includes, for example, a character input function such as a keyboard. The information input device 90 accepts a predetermined operation by the user and inputs various kinds of information, such as environmental data and track record information, to the training system 10 in accordance with the accepted operation. The information input device 90 may be a touch panel superimposed on the display device 91.

The display device 91, which includes, for example, a liquid crystal panel or an organic Electronic Luminescent (EL) panel, is able to present various kinds of information to the user. The display device 91 accepts information regarding the training plan from the plan generation unit 13 and displays the accepted information in an aspect in which the user can visually recognize the accepted information.

Note that the information input device 90 and the display device 91 may be configured integrally with the training system 10. Further, the information input device 90 and the display device 91 may each be a device including an information input/output function, such as a smartphone or a tablet that is separate from the training system 10, and may be connected to the training system 10 in such a way that they can communicate with each other.

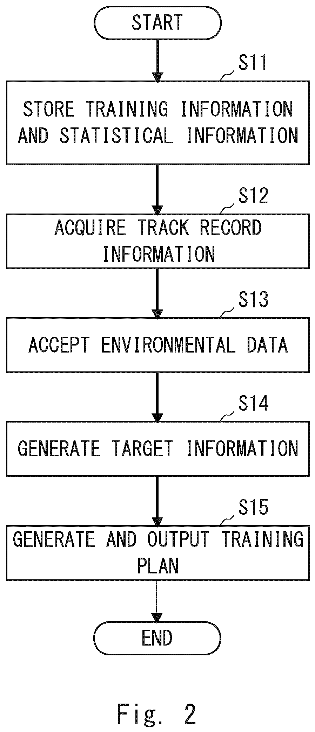

Referring next to Fig. 2, processing performed by the training system 10 will be described. Fig. 2 is a flowchart of the training system according to the first embodiment. First, the training system 10 stores the training information 141 and the statistical information 142 (Step S11). This processing is performed, for example, by the user supplying the aforementioned information to the training system 10 via the information input device 90.

Next, the track record information accepting unit 12 of the training system 10 acquires the track record information via the information input device 90 (Step S12). Next, the target information generation unit 11 accepts environmental data via the information input device 90 (Step S13). Further, the target information generation unit 11 generates target information from the accepted environmental data (Step S14).

Next, the plan generation unit 13 of the training system 10 generates a training plan corresponding to the accepted track record information and the generated target information. At this time, the plan generation unit 13 uses the training information and the statistical information stored in advance. The plan generation unit 13 outputs the generated training plan to the display device 91 (Step S15).

The configuration of the training system 10 and the processing performed by the training system 10 have been described above. As described above, the training system 10 accepts environmental data, and generates target information in accordance with an environment after the trainee finishes training. Then the training system 10 sets the generated target information as a target of the training, generates the training plan for the set target, and outputs the generated training plan.

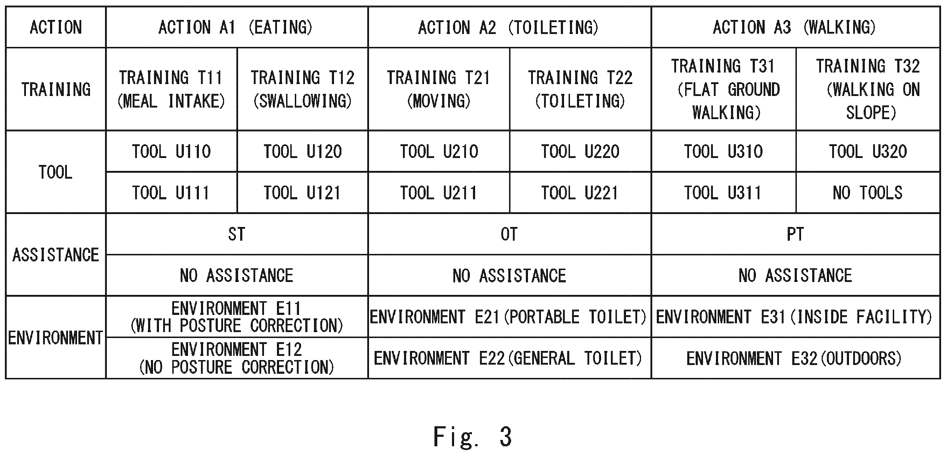

Next, specific examples of the training information 141 and therapist information 112 will be described with reference to Fig. 3. Fig. 3 is a diagram showing the training information according to the first embodiment. The table in Fig. 3 shows one example of information stored in the storage unit 14. Further, items listed in the table shown in Fig. 3 indicate possible combinations of training, tools, assistance, and environment that correspond to the respective physical actions.

The table shown in Fig. 3 shows, in the upper row, three physical actions including an action A1 (eating), an action A2 (toileting), and an action A3 (walking). Under the action A1, training T11 (meal intake) and training T12 (swallowing) are shown as training for recovering functions of the eating action. Under the training T11, tools used in the training T11 (tools U110 and U111) are shown. Further, under the training T12, tools used in the training T12 (tools U120 and U121) are shown. Further, under the column indicating tools, a therapist "ST" who assists the training T11 and the training T12 is shown as well as the description "no assistance". Under the column indicating assistance, an environment E11 (with posture correction) and an environment E12 (no posture correction) are shown. The environment E11 shows that posture correction is performed at the time of training. The environment E12 shows that the posture correction is not performed at the time of training.

The plan generation unit 13 is able to select the training T11 or the training T12 as training for the action A1. When the training T11 is performed, the plan generation unit 13 is able to select one or both of the tool U110 and the tool U111. When the training T12 is performed, the plan generation unit 13 is able to select one or both of the tool U120 and the tool U121. Likewise, when the training T11 or the training T12 is performed, the plan generation unit 13 is able to select whether or not the assistance should be provided by the ST. Further, when the training T11 or the training T12 is performed, the plan generation unit 13 is able to select the environment E11 or the environment E12.

Under the action A2, as training exercises for recovering functions of the toileting action, training T21 (moving) and training T22 (toileting) are shown. Under the training T21, tools used in the training T21 (tools U210 and U211) are shown. Further, under the training T22, tools used in the training T22 (tools U220 and U221) are shown. Further, under the column indicating tools, a therapist "OT" who assists the training T21 and the training T22 is shown as well as the description "no assistance". Further, under the column indicating assistance, an environment E21 (portable toilet) and an environment E22 (general toilet) are shown.

The plan generation unit 13 is able to select the training T21 or the training T22 as training for the action A2. Further, the plan generation unit 13 is able to select one or both of the tool U210 and the tool U211 when the training T21 is performed. The plan generation unit 13 is further able to select one or both of the tool U220 and the tool U221 when the training T22 is performed. Further, the plan generation unit 13 is able to select whether or not the assistance should be provided by the OT when the training T21 or the training T22 is performed. The plan generation unit 13 is able to select the environment E21 or the environment E22 when the training T21 or the training T22 is performed.

Under the action A3, training T31 (flat ground walking) and training T32 (walking on a slope) are shown as training for recovering functions of walking actions. Under the training T31, tools used in the training T31 (tools U310 and U311) are shown. Further, under the training T32, a tool used in the training T32 (tool U320) and "no tools" are shown. Further, under the column indicating tools, a therapist "PT" who assists the training T31 and the training T32 is shown as well as the description "no assistance". Further, under the column indicating assistance, an environment E31 (inside a facility) and an environment E32 (outdoors) are shown. Note that the aforementioned tools U310, U311, and U320 are, for example, a short leg brace, a long leg brace, a T-cane, and a four-leg stick.

The plan generation unit 13 is able to select the training T31 or the training T32 as training for the action A3. Further, the plan generation unit 13 is able to select one or both of the tool U310 and the tool U311 when the training T31 is performed. Further, the plan generation unit 13 is able to select the tool U320 or no tools when the training T32 is performed. Further, the plan generation unit 13 is able to select whether or not the assistance should be provided by the PT when the training T31 or the training T32 is performed. The plan generation unit 13 is able to select the environment E31 or the environment E32 when the training T31 or the training T32 is performed.

Specific examples of the training information 141 have been described above. As described above, the storage unit 14 stores information including the training information 141. The plan generation unit 13 selects the training for each action from combinations in the table shown in Fig. 3. The information shown in the table in Fig. 3 is merely one example for describing this embodiment and is not intended to limit the content of the training information 141 and the therapist information 112. For example, items regarding the actions are not limited to the actions A1 to A3. Further, the training, the tools, the assistance, and the environment in each action are not limited to the above content as well.

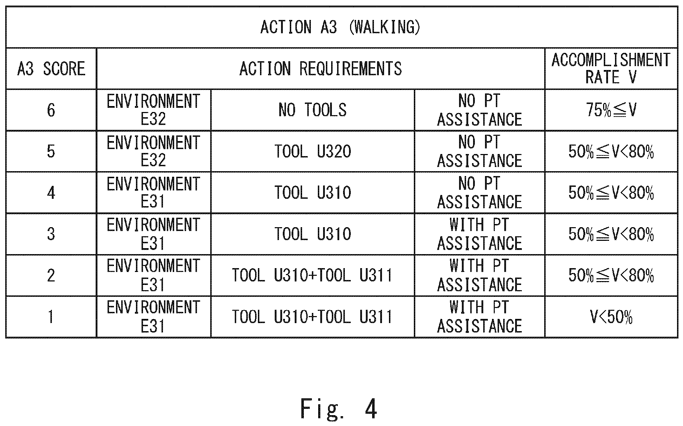

Referring next to Fig. 4, action levels will be described. Fig. 4 is a diagram showing an action level according to the first embodiment. The training system 10 uses the action level described below. The action level is an index indicating the level of the physical action set using at least information regarding the type of the tool that the trainee uses in the physical action and the level of achievement of the physical action.

The table shown in Fig. 4 shows, as one example of action levels in the physical actions, an action level related to the action A3 (walking). The left-side column in the table shown in Fig. 4 shows six stages from 1 to 6 as an "A3 score". Further, the table shown in Fig. 4 shows action requirements in a case in which the A3 score is 1 to 6, and the accomplishment rate V. The action requirements include the environment, and the presence or the absence of assistance by tools and the PT. The accomplishment rate V of the physical action can be determined, for example, from signals from a posture detection sensor which is worn by the trainee.

Assume a case, for example, the A3 score is determined to be 1. The trainee performs a predetermined action in the environment E31, using the tools U310 and U311, and with the assistance provided by the PT. In this case, when the achievement rate V of an action is smaller than 50 percent, the A3 score becomes 1. Further, as shown in the second row from the bottom in Fig. 4, when the achievement rate of the action is 50 percent or larger but smaller than 80 percent in the aforementioned action requirements, the A3 score becomes 2.

Further, as the action requirements, when a trainee performs a predetermined action in the environment E31, using the tool U310, and with the assistance of the PT and the achievement rate V of the action is 50 percent or larger but smaller than 80 percent, then the A3 score becomes 3. As the action requirements, when a trainee performs a predetermined action in the environment E31, using the tool U310, and without the assistance of the PT and the achievement rate V of the action is 50 percent or larger but smaller than 80 percent, then the A3 score becomes 4. Further, as the action requirements, when a trainee performs a predetermined action in the environment E32, using the tool U320, and without the assistance of the PT and the achievement rate V of the action is 50 percent or larger but smaller than 80 percent, then the A3 score becomes 5. As the action requirements, when a trainee performs a predetermined action in the environment E32, using the tool U320, and without the assistance of the PT and the achievement rate V of the action is 75 percent or larger, then the A3 score becomes 6.

One example of the action levels has been described above. The action level is composed of objective information, not based on subjective judgment. The training system 10 receives information regarding the action level of the trainee from the user by using this action level. Accordingly, the user is able to input track record information regarding physical actions of the trainee in a simple way. Further, the training system 10 is able to set a target of a training plan using the action level. The training system 10 is also able to generate the training plan using the action level. Therefore, the training system 10 is able to present the training plan to the user in an aspect in which the user can easily understand the training plan using the action level and can evaluate the training plan objectively.

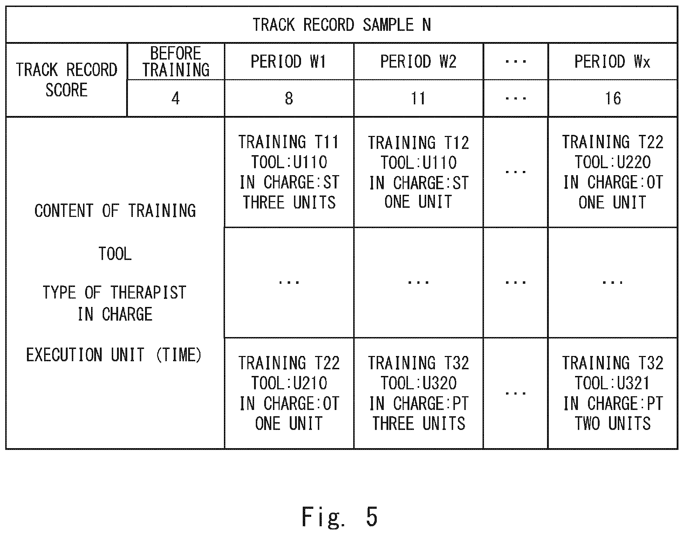

Referring next to Fig. 5, the statistical information will be described. Fig. 5 is a diagram showing the statistical information according to the first embodiment. The table in Fig. 5 shows an extraction of a track record sample N, which is the N-th sample included in the statistical information. The track record sample N is a piece of sample information that composes the statistical information. That is, the statistical information includes a plurality of track record samples as shown in the table in Fig. 5. The track record sample N includes information in which the content of the training that a predetermined trainee has performed, the type of the therapist who is in charge of the training, and the degree of recovery of the physical action of the trainee as a result of the training performed are associated with one another.

The table in Fig. 5 shows, in the upper row, that the track record sample is N. The table in Fig. 5 further shows, in the second row from the top, the track record score. The track record score is an action level which indicates the past records of the trainee in the track record sample N. In the track record score, the action level before the training and a transition of the action level from the first period W1 to the x-th period Wx are shown. The period W1 is, for example, a period in which one week is set as one unit. That is, the period W1 means the first week, the period W2 means the second week, and the period Wx means the x-th week. For the sake of convenience of explanation, the periods W3 to Wx₋₁ and some columns indicating the details of the training are omitted.

In the row under the track record score, details of the training carried out in each period are shown. The details of the training include the content of the training, tools used in the training, the type of the therapist who is in charge, and the execution unit (time). The execution unit indicates a time period during which training is performed. For example, one unit is set as 30 minutes.

In the track record sample N shown in the table in Fig. 5, the track record score of the trainee before the training is 4. Further, the track record level after the period W1 is ended is 8. In the period W1, for example, the training T11 and the training T22 are performed. The table in Fig. 5 shows that the training T11 performed in the period W1 uses the tool U110, the therapist ST is in charge, and the three units (that is, 90 minutes) of training have been performed. The table in Fig. 5 further shows that the training T22 performed in the period W1 uses the tool U210, the therapist who is in charge of this training is OT, and the execution unit is one unit.

Likewise, the track record sample N shown in the table in Fig. 5 shows that the track record level after the period W2 is ended is 11. According to the table shown in Fig. 5, the training T12 and the training T32 are performed in the period W2. The tool used in the training T12 in the period W2 is the tool U110, the therapist who is in charge of this training is an ST, and the execution unit is one unit. The tool used in the training T32 in the period W2 is the tool U320, the therapist who is in charge of this training is a PT, and the execution unit is three units. Further, according to the table shown in Fig. 5, the training T22 and the training T32 are performed in the period Wx. The tool used in the training T22 in the period Wx is the tool U220, the therapist who is in charge of this training is an OT, and the execution unit is one unit. The tool used in the training T32 in the period Wx is the tool U321, the therapist who is in charge of this training is a PT, and the execution unit is two units.

As described above, the track record sample includes, for training performed by a predetermined trainee, tools that have been used, the therapist who is in charge, or a transition of the physical action level. It can also be said that the transition of the physical action level indicates the trainee's degree of recovery. The plan generation unit 13 generates the training plan using statistical information including a plurality of track record samples. Accordingly, the training system 10 is able to generate the training plan which enables the level of the trainee's physical action level to be efficiently improved.

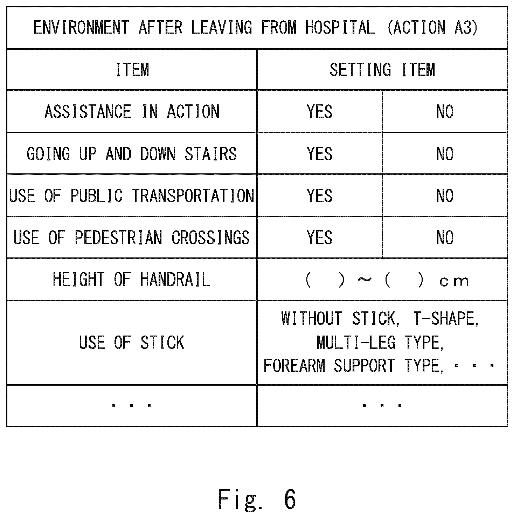

Next, the environmental data will be described with reference to Fig. 6. Fig. 6 is a diagram showing an example of the environmental data of the training system according to the first embodiment. The table shown in Fig. 6 is an example of the environmental data of the action A3 (walking). The left column of the table in Fig. 6 shows items regarding the environment of a trainee. Further, the right column of the table in Fig. 6 shows setting items corresponding to the settings of the respective items regarding the environment.

Specifically, for example, an item "assistance in action" is shown at the top of the list of the items regarding the environment. Further, in the setting item corresponding to the assistance in action, "Yes" and "No" are shown. When the trainee who has finished the training plan receives the assistance in the action in his/her daily life, a user selects "Yes". On the other hand, when the trainee who has finished the training plan does not receive the assistance in the action in his/her daily life, the user selects "No".

An item "going up and down stairs" is shown under the item "assistance in action". The user indicates whether or not the trainee goes up and down the stairs in his/her daily life after the training by selecting "Yes" or "No" shown in the setting item. Likewise, regarding items "use of public transportation" and "use of pedestrian crossings", the user selects "Yes" or "No" shown in the respective setting items.

An item "height of handrail" is shown under the item "use of pedestrian crossings". The user inputs a specific range of the height of the handrail as a setting item corresponding to the item "height of handrail". An item "use of stick" is shown under the item "height of handrail". The "use of stick" is data regarding the tools to be used by the trainee after he/she finishes the training plan. In the setting item corresponding to the "use of stick", "without stick, T-shape, multi-leg type, forearm support type" and the like are shown. The user selects whether or not the trainee uses a stick as a setting item corresponding to the item "use of stick". Further, when the trainee uses a stick, the user selects it from among a T-shape, a multi-leg type, a forearm support type, and the like.

The training system 10 receives information about an environment of the trainee after he/she finishes the training plan by using the data shown in Fig. 6. That is, the training system 10 presents the table shown in Fig. 6 and prompts the user to input environmental data. The user who inputs the environmental data selects or inputs the environmental data for each item in the presented table.

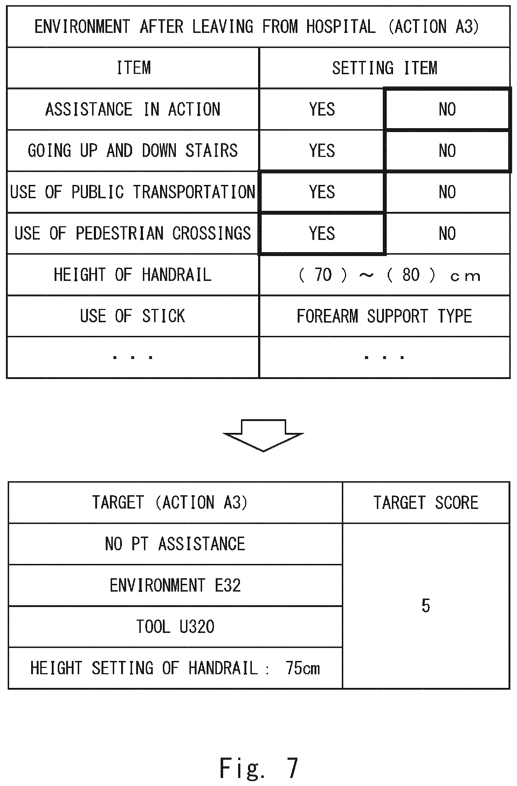

Next, the target information will be described with reference to Fig. 7. Fig. 7 is a diagram showing an example of the target information generated from the accepted environmental data. The table in the upper part of Fig. 7 shows a state in which a user to whom the environmental data shown in Fig. 6 has been presented has selected or input the setting item of each item. In the table in the upper part of Fig. 7, "No" is selected for "assistance in action". Similarly, "No" is selected for "going up and down stairs", "Yes" is selected for "use of public transportation", and "Yes" is selected for "use of pedestrian crossings". The item "height of handrail" indicates a range of "70 to 80" cm, which has been input by the user. Further, the "forearm support type" is selected for the item "use of stick".

The table in the lower part of Fig. 7 shows the target information generated by the target information generation unit 11 from the environmental data shown in the upper part thereof. In the table in the lower part of Fig. 7, the left column shows the target information generated by the target information generation unit 11 with regard to the walking action. The target information shown here corresponds to the environmental data shown in the upper part of Fig. 7. That is, in the environmental data, since "No" has been selected for the assistance in action, the target information includes information indicating "no PT assistance". In other words, the target of the training plan carried out by the trainee concerning the information shown in Fig. 7 is that the trainee is able to walk without the assistance of the PT eventually.

In the table in the lower part of Fig. 7, pieces of information indicating the "environment E32", the "tool U320", and a "height setting of handrail: 75 cm" are shown under "no PT assistance". These pieces of information correspond to the environmental data shown in the upper part of Fig. 7. Further, these pieces of information indicate that a target of the training of the walking action is that the trainee is able to walk using "the tool U320" in "the environment E32" under a condition that "the height setting of a handrail is 75 cm".

In the table in the lower part of Fig. 7, an item "target score" is shown in the right column, and an action score "5" as target information is shown under the "target score". The target information generation unit 11 sets each item on the left side of the table shown in the lower part of Fig. 7 based on the table shown in the upper part thereof, and selects and displays the action score "5" corresponding to each item on the left side of Fig. 7 as a target action score.

The target information has been described above. As described above, the target information generation unit 11 generates the target information from the received environmental data. The target information generation unit 11 may store in advance reference data for associating the environmental data with the target information. Further, the target information generation unit 11 may include a learned model for generating the target information from information that is accepted as the environmental data. In this case, the target information generation unit 11 may input the accepted environmental data to the learned model and use information acquired in response to this input as the target information.

Note that although an example using the action A3, which is a walking action, has been described above, the target information of the walking action is not limited to the items described above. Further, although the target information is generated for actions other than the walking action based on the above-described principle, the target information generated for the actions other than the walking action is not limited to the information shown in Fig. 7.

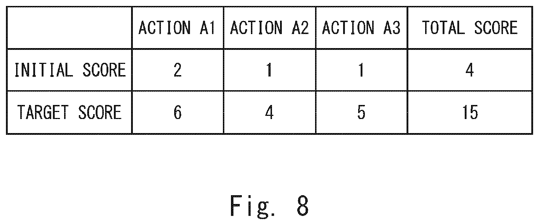

Next, track record information and target information will be described with reference to Fig. 8. Fig. 8 is a diagram showing the track record information and the target information according to the first embodiment. The table shown in Fig. 8 shows initial scores and target scores of the action A1, the action A2, and the action A3. The right column in the table shown in Fig. 8 shows a total score obtained by adding the action scores of the action A1, the action A2, and the action A3 for each of the initial score and the target score.

The initial score is a score indicating the action level of the trainee just before the trainee starts training. The table shown in Fig. 8 shows that the initial score of the action A1 is 2, the initial score of the action A2 is 1, and the initial score of the action A3 is 1. The track record information accepting unit 12 acquires the above initial scores as the track record information of the trainee.

The target score is a score which corresponds to the action level in each action and which is generated by the target information generation unit 11. The table in Fig. 8 shows that the target score of the action A1 is 6, the target score of the action A2 is 4, and the target score of the action A3 is 5. When the target information generation unit 11 generates a target score as target information, the plan generation unit 13 receives the target score from the target information generation unit 11.

By using the initial score and the target score shown in Fig. 8, the plan generation unit 13 generates a training plan so that the trainee having the acquired initial score is able to achieve the target score by carrying out the training plan. At this time, the plan generation unit 13 generates the training plan by using the training information 141 and the statistical information 142 stored in the storage unit 14.

Note that, in the training system 10, the track record information accepting unit 12 may further acquire profile information of the trainee in addition to the initial score shown in Fig. 8. Accordingly, the training system 10 is able to generate the training plan in accordance with the trainee's profile.

Examples of the profile information will be described here. The profile information, which is information indicating information regarding a trainee, includes at least one of the following information (1) to (4).

### (1) Information indicating attributes of trainee

The information indicating attributes of the trainee includes, for example, the trainee's age, sex, physique (height, weight, etc.), and a score indicating the trainee's physical condition.

### (2) Symptom information of disease that trainee is suffering from

Symptom information may include a type(s) of a disease(s) (a name(s) of a disease(s) or a disorder(s)) that the subject has suffered from, such as a stroke (a cerebrovascular disorder) and a spinal cord injury. Further, the symptom information may also include, depending on the type of the disease, its classification. For example, strokes can be classified into cerebral infarction, intracranial hemorrhage (cerebral hemorrhage/subarachnoid hemorrhage), etc. The symptom information may also include information indicating an initial symptom, a time when the symptom appears, and a current symptom in association with the above information. The symptom information may also include symptoms that are unlikely to be directly related to the rehabilitation in addition to the information indicating that the trainee needed to perform rehabilitation because of the symptoms included in the symptom information.

### (3) Trainee's assessment score based on stroke impairment assessment set

There is, for example, Stroke Impairment Assessment Set (SIAS) as an assessment method for quantifying an index for dysfunction caused by a stroke the trainee is suffering from. The SIAS may include a hip flexion test (Hip-Flex), a knee extension test (Knee-Ext), and a foot-pat test (Foot-Pat). Further, the SIAS may also include a lower limb tactile sensation (Touch L/E), a lower limb position sensation (Position L/E), an abdominal muscle strength (Abdominal), and a verticality test (Verticality).

### (4) Information indicating trainee's degree of recovery

The transition of the action level described above may be employed as the information indicating the trainee's degree of recovery. Specifically, when a certain amount of time has passed immediately after the trainee gets a disease such as a hemiplegia, changes in the action level during this period may be information indicating the trainee's degree of recovery. Further, the information indicating the trainee's degree of recovery may be the Brunnstrom Recovery Stage (Br.stage). The Brunnstrom Recovery Stage (Br.stage) is an indicator of recovery in which a recovery process of a hemiplegia is divided into six stages based on the observation. In this embodiment, the information indicating the trainee's degree of recovery may include, of the Br.stage, lower-limb items that are main items related to the walking training apparatus 100.

Although the profile information has been described in detail above, the profile information is not limited to the above-described items and may be information of types different from those described above as long as it includes information satisfying the purpose of the profile information. The profile information may also include additional information such as a date and a time when information is measured.

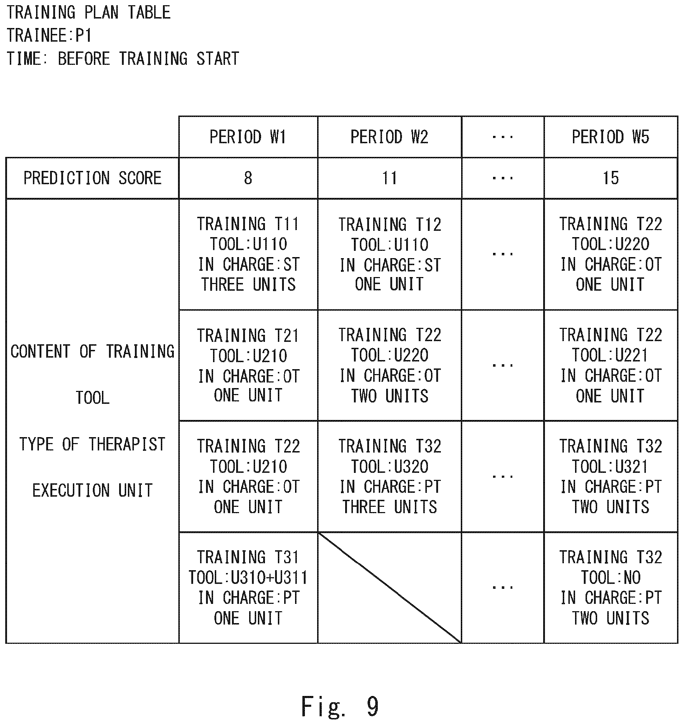

Next, the training plan generated by the plan generation unit 13 will be described with reference to Fig. 9. Fig. 9 is a first diagram showing the training plan according to the first embodiment. Fig. 9 shows the training plan generated by the plan generation unit 13 that has acquired the track record information and the target information shown in Fig. 8. The training plan shown in the table in Fig. 9 is the one of a trainee PI, and the time is before the start of the training.

In the table shown in Fig. 9, the upper row shows periods W1 to W5. Under the periods W1 to W5, prediction scores are shown. The prediction scores are the predicted action levels of the trainee after the training in periods indicated above the column indicating the respective numerical values is ended. The table in Fig. 9 shows that the action level after the period W1 is ended is 8, the action level after the period W2 is ended is 11, and the action level after the period W5 is ended is 15. For the sake of convenience of explanation, the periods W3 and W4 are omitted in the table in Fig. 9.

Under the column of the prediction scores, details of the training planned in each period are shown. Specifically, training exercises planned in the period W1 are the training T11, the training T21, the training T22, and the training T31. The training T11 uses the tool U110, the therapist ST is in charge, and the execution unit is three units. The training T21 uses the tool U210, the therapist OT is in charge, and the execution unit is one unit. The training T22 uses the tool U210, the therapist OT is in charge, and the execution unit is one unit. The training T31 uses the tools U310 and U311, the therapist PT is in charge, and the execution unit is one unit.

The training exercises planned in the period W2 are training T12, training T22, and training T32. The training T12 uses the tool U110, the therapist ST is in charge, and the execution unit is one unit. The training T22 uses the tool U220, the therapist OT is in charge, and the execution unit is two units. The training T32 uses the tool U320, the therapist PT is in charge, and the execution unit is three units.

The training exercises planned in the period W5 are training T22, training T22, training T32, and training T32. One training T22 uses the tool U220, the therapist OT is in charge, and the execution unit is one unit. The other training T22 uses the tool U221, the therapist OT is in charge, and the execution unit is one unit. One training T32 uses the tool U321, the therapist PT is in charge, and the execution unit is two units. The other training T32 does not use any tools, the therapist PT is in charge, and the execution unit is two units.

As described above, the plan generation unit 13 generates the content of the training for each period, the tool to be used, the type of the therapist who is in charge, and the execution unit, which is a time when the training is executed. The plan generation unit 13 combines pieces of information in the training information shown in Fig. 3 with each other, thereby generating the training plan shown in the table in Fig. 9. In this way, the plan generation unit 13 generates a training plan from the track record information, the target information, the training information, and the statistical information. Further, the plan generation unit 13 indicates the prediction score using the action level, whereby it is possible for the training system 10 to output a training plan using an index which a user can easily understand.

The first embodiment has been described above. The training plan generated by the training system 10 according to the first embodiment is not limited to the aforementioned one related to the physical actions. The physical actions regarding which the training plan is generated include various other actions related to ADL. Further, while the PT, the OT, and the ST have been mentioned as the therapists in the above-described description, a person who is in charge of training or a person who assists the trainee in the physical actions may include, for example, a nurse in a medical institute or a family member of the trainee. The number of training exercises related to the physical actions according to this embodiment is not limited as long as it is plural. The plan generation unit 13 may generate a plurality of different training plans that can be executed and output the generated plans. Accordingly, the training system is able to present a plurality of training plans that can be selected to the user.

As described above, the training system 10 is able to set a target in accordance with an environment after the trainee finishes training, and output the training plan for the set target. Therefore, according to the first embodiment, it is possible to provide a training system and the like that enable a trainee to perform function recovery training efficiently.

### <Second Embodiment>

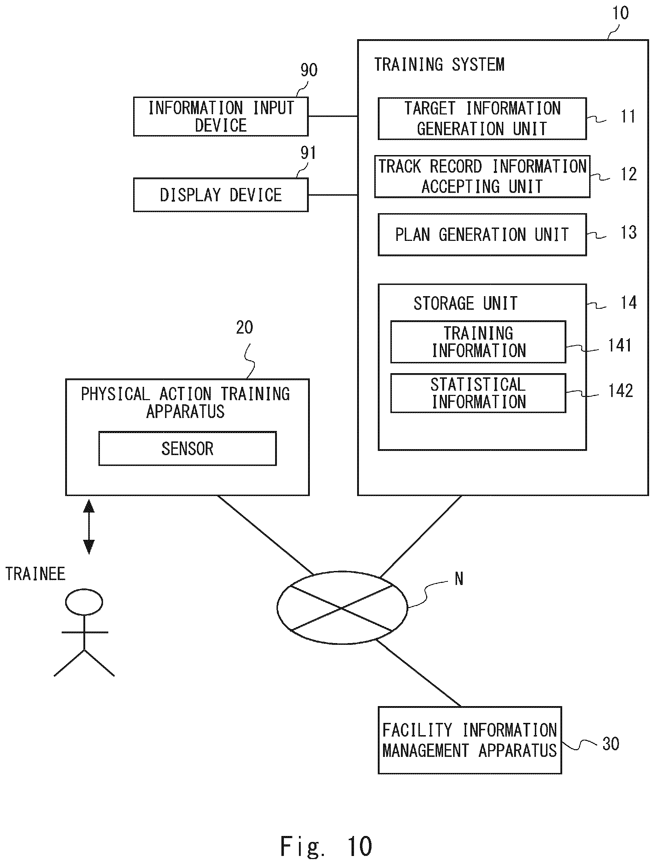

Next, a second embodiment will be described. The second embodiment differs from the first embodiment in that various kinds of information are acquired via a network. Fig. 10 is a block diagram of a training system according to the second embodiment. The training system 10 according to the second embodiment is connected to a physical action training apparatus 20 and a facility information management apparatus 30 via a network N so that they communicate with one another. The network N is a communication line network such as the Internet, an intranet, a mobile phone network, and a Local Area Network (LAN).

The physical action training apparatus 20 is an apparatus for training the physical actions of a trainee. The physical action training apparatus 20 may be a walking apparatus, such as a treadmill, or may be an apparatus that detects or measures the physical actions of the trainee. The physical action training apparatus 20 includes at least a sensor that acquires information regarding the physical actions of the trainee.

The sensor may be a contact-type sensor worn and used by the trainee or may be a non-contact sensor used by the trainee without wearing it. The contact-type sensor is, for example, a pressure sensor, an acceleration sensor, an angle sensor etc. Further, the non-contact sensor is, for example, an image sensor, an infrared sensor, a distance measurement sensor etc.

The track record information accepting unit 12 may acquire image information from a camera that has captured physical actions of a trainee as the track record information. In this case, the training system 10 is able to recognize, from the acquired image information, the state of the physical actions performed by the trainee and thereby determine the levels of achievement of these physical actions. Accordingly, the training system 10 is able to easily acquire the track record information.

The physical action training apparatus 20 performs training of the trainee for the physical actions and acquires information regarding the physical actions in the training. The physical action training apparatus 20 supplies the acquired information to the training system 10 via the network N.

The facility information management apparatus 30 has environmental data of a trainee in a facility where the trainee will spend his/her daily life after he/she finishes the training plan. For example, it is assumed that the trainee performs training for the physical action at a predetermined hospital and will spend his/her daily life in a predetermined medical care facility after he/she finishes the training plan. In this case, the facility information management apparatus 30 stores information about equipment and the like in the predetermined medical care facility. The equipment and the like in the aforementioned predetermined medical care facility are handrails, stairs, ramps, elevators, toilets, and the like in this medical care facility. The facility information management apparatus 30 stores information indicating, for example, the height in centimeters of the handrail of the medical care facility, whether or not the trainee has to pass over a step in the medical care facility, and whether or not the trainee who is in a wheelchair can use the toilet equipment of the medical care facility by himself/herself. The facility information management apparatus 30 is connected to the training system 10 so that they communicate with each other, and provides the above-described information in response to a request from the training system 10.

In this embodiment, for example, the target information generation unit 11 accepts, from the information input device 90, information indicating that the trainee will spend his/her daily life in the medical care facility after he/she finishes the training plan. Then the target information generation unit 11 accesses the facility information management apparatus 30 and receives information regarding the equipment of the medical care facility as environmental data. Further, the target information generation unit 11 generates target information from the information received as the environmental data.

The second embodiment has been described above. The training system 10 according to the second embodiment acquires various kinds of information via the network N. Therefore, it is possible to reduce the burden on a user inputting information by a manual operation and to efficiently acquire information. Further, it is possible for the training system 10 to appropriately acquire desired information.

As described above, the training system 10 is able to set a target in accordance with an environment after a trainee finishes training, and output the training plan for the set target. Therefore, according to the second embodiment, it is possible to provide a training system and the like that enable a trainee to perform function recovery training efficiently.

Note that the present disclosure is not limited to the above embodiments and may be changed as appropriate without departing from the spirit of the present disclosure. The program can be stored and provided to a computer using any type of non-transitory computer readable media. Non-transitory computer readable media include any type of tangible storage media. Examples of non-transitory computer readable media include magnetic storage media (such as floppy disks, magnetic tapes, hard disk drives, etc.), optical magnetic storage media (e.g., magneto-optical disks), CD-ROM (compact disc read only memory), CD-R (compact disc recordable), CD-R/W (compact disc rewritable), and semiconductor memories (such as mask ROM, PROM (programmable ROM), EPROM (erasable PROM), flash ROM, RAM (random access memory), etc.). The program may be provided to a computer using any type of transitory computer readable media. Examples of transitory computer readable media include electric signals, optical signals, and electromagnetic waves. Transitory computer readable media can provide the program to a computer via a wired communication line (e.g., electric wires, and optical fibers) or a wireless communication line.

From the disclosure thus described, it will be obvious that the embodiments of the disclosure may be varied in many ways. Such variations are not to be regarded as a departure from the spirit and scope of the disclosure, and all such modifications as would be obvious to one skilled in the art are intended for inclusion within the scope of the following claims.

## Claims

1. A training system (10) configured to generate a training plan for enabling a trainee to perform a plurality of kinds of training for the purpose of recovering a function of a physical action of the trainee, the training system comprising:
a track record information accepting unit (12) configured to accept track record information regarding the physical action of the trainee;
a target information generation unit (11) configured to accept environmental data of daily life of the trainee after the training plan is finished, and generate target information regarding a target of the function recovery of the trainee based on the accepted environmental data;
a storage unit (14) configured to store statistical information regarding the function recovery in the training performed by a plurality of other trainees; and
a plan generation unit (13) configured to generate the training plan of the trainee based on the track record information, the target information, and the statistical information and output the generated training plan.

2. The training system (10) according to Claim 1, wherein the target information generation unit acquires, by it being connected to a management apparatus configured to manage the environmental data, the environmental data from the management apparatus.

3. The training system (10) according to Claim 1 or 2, wherein
the storage unit (14) includes, in the environmental data, data regarding a tool used by the trainee in the physical action, and
the target information generation unit (11) presents a candidate for the tool to be used by the trainee after the training plan is finished based on the environmental data.

4. The training system (10) according to Claim 3, wherein the plan generation unit (13) determines the tool used by the trainee in the training plan based on the tool to be used by the trainee after the training plan is finished.

5. The training system (10) according to any one of Claims 1 to 4, wherein the track record information accepting unit (12) acquires, as the track record information, information from a sensor that has detected the physical action of the trainee.

6. The training system (10) according to any one of Claims 1 to 4, wherein the track record information accepting unit (12) acquires, as the track record information, image information from a camera that has captured images of the physical action of the trainee.

7. The training system (10) according to any one of Claims 1 to 6, wherein the track record information accepting unit (12) acquires information regarding an action level of the trainee set using at least information regarding a type of the tool used by the trainee in the physical action and a level of achievement of the physical action, and
the plan generation unit generates the training plan based on the action level.

8. The training system (10) according to Claim 7, wherein the target information generation unit (11) generates the action level as the target information based on the environmental data.

9. A training method for generating a training plan for enabling a trainee to perform a plurality of kinds of training for the purpose of recovering a function of a physical action of the trainee, the training method comprising:
a track record information accepting step of acquiring track record information regarding the physical action of the trainee;
a target information generation step of accepting environmental data of daily life of the trainee after the training plan is finished, and generating target information regarding a target of the function recovery of the trainee based on the accepted environmental data;
a storage step of storing statistical information regarding the function recovery in the training performed by a plurality of other trainees; and
a plan generation step of generating the training plan of the trainee based on the track record information, the target information, and the statistical information and outputting the generated training plan.

10. A program for causing a computer to execute a training method for generating a training plan for enabling a trainee to perform a plurality of kinds of training for the purpose of recovering a function of a physical action of the trainee, the training method comprising:
a track record information accepting step of acquiring track record information regarding the physical action of the trainee;
a target information generation step of accepting environmental data of daily life of the trainee after the training plan is finished, and generating target information regarding a target of the function recovery of the trainee based on the accepted environmental data;
a storage step of storing statistical information regarding the function recovery in the training performed by a plurality of other trainees; and
a plan generation step of generating the training plan of the trainee based on the track record information, the target information, and the statistical information and outputting the generated training plan.
